# EUROPÄISCHE PATENTANMELDUNG

(11) **EP 1 992 284 A1**
(43) Veröffentlichungstag der Anmeldung: **19.11.2008**
(21) Anmeldenummer: 07108252.3
(22) Anmeldetag: 15.05.2007
(51) Int. Cl.: A61B 5/151

(54) **Verfahren zur Magazinierung von Stechelementen und Bandmagazin**

(71) Anmelder: F.HOFFMANN-LA ROCHE AG, 4070 Basel (CH); Roche Diagnostics GmbH, 68305 Mannheim (DE)
(72) Erfinder: Deck, Frank, D-67150 Niederkirchen (DE); Hiller, Bernd, D-68623 Lampertheim (DE); Konya, Ahmet, D-67165 Waldsee (DE); Zimmer, Volker, D-67229 Laumersheim (DE)
(74) Vertreter: Pfiz, Thomas

(57) **Zusammenfassung**

Die Erfindung betrifft ein Verfahren zur Magazinierung von Lanzetten sowie ein entsprechendes Verfahrenserzeugnis, bei dem eine Vielzahl von mit einer Lanzettenspitze (28) zum Einstechen in die Haut versehenen disposiblen Lanzetten (12) auf einem auf einer Spule (60,62) aufgewickelten oder einem aufwickelbaren Trägerband (18) zum Gewinnen einer Körperflüssigkeitsprobe bereitgestellt werden. Erfindungsgemäß wird vorgeschlagen, dass die Lanzetten (12) in jeweils einer Packung (14) angeordnet werden, und dass die so gebildeten Lanzetten-Packungen (16) einzeln auf das Trägerband (18) aufgebracht und darauf fixiert werden.

## Beschreibung

Die Erfindung betrifft ein Verfahren zur Magazinierung von Lanzetten und ggf. Testelementen insbesondere für Blutglucoseuntersuchungen bei dem eine Vielzahl von mit einer Lanzettenspitze zum Einstechen in die Haut versehenen disposiblen Lanzetten auf einem auf einer Spule aufgewickelten oder einem aufwickelbaren Trägerband zum Gewinnen einer Körperflüssigkeitsprobe bereitgestellt werden. Die Erfindung betrifft weiter ein durch ein solches Verfahren hergestelltes Bandmagazin.

Aus der WO-A 2005/107596 ist eine Anordnung von Lanzetten auf einem Trägerband bekannt, wobei sich die einzelnen Lanzetten durch Bandtransport sukzessive zum Einsatz bringen und auch wieder zu entsorgen lassen. Die Lanzetten sollen im Zuge der Fertigung durch eine direkte Verbindung auf dem Band integriert werden. Daneben ist auch eine zusätzliche Bereitstellung eines Testmediums auf dem Band offenbart. Damit kann dem Benutzer die Handhabung bei der Durchführung von Selbsttests insbesondere für die Blutzuckerüberwachung erleichtert werden.

Ausgehend hiervon liegt der Erfindung die Aufgabe zugrunde, die im Stand der Technik bekannten Fertigungsverfahren und Verfahrenserzeugnisse weiter zu verbessern und so zu gestalten, dass eine günstige Massenproduktion bei hoher Produktqualität möglich ist. Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, die disposiblen Elemente gesondert auf einem ersten Träger zu verpacken und dann etikettenartig auf einem zweiten Träger zu applizieren. Dementsprechend wird erfindungsgemäß vorgeschlagen, dass die Lanzetten in jeweils einer Packung angeordnet werden, und dass die so gebildeten Lanzetten-Packungen einzeln auf das Trägerband aufgebracht und darauf fixiert werden. Auf diese Weise ergeben sich für die Magazinierung steril zu haltender Stechelemente besondere Vorteile. Die Vorverpackung erlaubt einen Sterilschutz auch in einer Fertigungsumgebung und vereinfacht zugleich die maschinelle Handhabung bei der Verbindung mit dem Trägerband. Zudem ergibt sich durch die Verpackung auch eine erhöhte Lagerstabilität.

Um eine flache, aufrollbare Struktur zu schaffen, ist es vorteilhaft, wenn die Packungen aus einer Bodenfolie und einer damit verbundenen Deckfolie gebildet werden. Hierbei können die Bodenfolie und die Deckfolie unter Einschluss der Lanzetten miteinander in Kontakt gebracht und verbunden werden.

Zusätzliche Bauteile lassen sich dadurch vermeiden, dass die Bodenfolie und die Deckfolie über eine Schmelzkleberverbindung miteinander verbunden werden, wobei eine vorzugsweise auf der Bodenfolie befindliche Schmelzkleberschicht durch einen Heißstempel aktiviert wird.

Herstellungsvorteile ergeben sich dadurch, dass eine Vielzahl von Lanzetten-Packungen als Packungsband zusammenhängend gefertigt und vor dem Aufbringen auf das Trägerband vereinzelt wird. Eine weitere fertigungstechnische Vereinfachung sieht vor, dass das Packungsband durch Zusammenführen und Verbinden eines Bodenfolienstreifens und eines Deckfolienstreifens gebildet wird. Alternativ ist es auch möglich, dass das Packungsband durch Umklappen eines Folienstreifens und Bildung von Lanzettentaschen in dem gefalteten Folienstreifen gefertigt wird.

Eine Sterilverpackung lässt sich dadurch erreichen, dass die Packungen durch einen Heißschnitt zugleich zugeschnitten und unter Einschluss der Lanzetten dicht umrandet werden.

Um eine gerätetechnische Handhabung zu vereinfachen, ist es vorteilhaft, wenn die Lanzetten vorzugsweise an einem proximalen Endabschnitt fest mit der Packung und vorzugsweise mit der Bodenfolie verbunden werden.

Eine besonders bevorzugte Ausgestaltung sieht vor, dass zumindest Spitzen der Lanzetten in der Packung gegen Beschädigung geschützt und/oder steril gehalten sind.

Vorteilhafterweise werden die Lanzettenspitzen in einer durch die Packung gebildeten Tasche angeordnet. Denkbar ist es auch, dass die Packungen durch eine Bodenfolie und eine darauf aufgebrachte und zumindest die Lanzettenspitze umschließende, insbesondere aus Silikon oder TPE bestehende biokompatible Verschlussmasse gebildet werden. In jedem Fall sollte die Tasche bzw. der Verschlussblock leicht penetrierbar sein, um die Lanzettenspitze für den Gebrauch freizugeben.

Eine weitere vorteilhafte Ausgestaltung besteht darin, dass zumindest die Spitzen der Lanzetten in den Lanzetten-Packungen vor dem Aufbringen auf das Trägerband vorzugsweise durch hochenergetische Bestrahlung sterilisiert werden. Damit lässt sich sowohl ein gezielter Sterilschutz einer hohen Stückzahl als auch eine nachfolgende Handhabung der Lanzetten-Packungen außerhalb eines Sterilbereichs realisieren.

Die Applikation auf das Trägerband lässt sich dadurch einfach bewerkstelligen, dass die Lanzetten-Packungen jeweils an einer von der Lanzette abgewandten Verbindungsfläche auf dem Trägerband fixiert werden. Besonders bevorzugt werden die Lanzetten-Packungen in Form von Etiketten oder Aufklebern auf das Trägerband aufgebracht. Hierbei ist es günstig, wenn die Lanzetten-Packungen mit einem Klebebandstück zum Aufkleben auf das Trägerband versehen werden.

Grundsätzlich ist es auch möglich, dass die Lanzetten-Packungen durch mechanische Verbindungsmittel oder durch Verschweißen (z.B. mittels Ultraschall oder Laser) auf dem Trägerband gehalten werden.

Eine besonders bevorzugte Variante der Erfindung sieht vor, dass auf dem Trägerband vorzugsweise alternierend zu den Lanzetten-Packungen mit der Köperflüssigkeitsprobe beaufschlagbare Testelemente aufgebracht werden. Dementsprechend wird vorgeschlagen, dass die Lanzetten und die Testelemente gesondert als Packungseinheiten vorgefertigt werden, und dass anschließend in einem einheitlichen Bestückungsprozess die Packungseinheiten auf das Trägerband aufgebracht und darauf fixiert werden. Auf diese Weise lassen sich völlig unterschiedliche disposible Einheiten beim Aufbringen auf das Band einheitlich handhaben. Dabei kann durch die gleichartige, aber separat durchgeführte Vorverpackung unterschiedlichen Anforderungen hinsichtlich Sterilschutz der Lanzetten und Schutz der Testchemie in besonderem Maß Rechnung getragen werden.

Eine weitere Fertigungsvereinfachung ergibt sich dadurch, dass die flachen Lanzetten- und Testelement-Packungseinheiten eine im wesentlichen übereinstimmende, vorzugsweise der Breite des Trägerbands entsprechende Breite aufweisen.

Weitere Gebrauchsvorteile lassen sich auch dadurch erzielen, dass das Trägerband in einer Kassette angeordnet wird, so dass es von einer Abwickelspule abziehbar und auf eine Aufwickelspule aufspulbar ist.

Im Hinblick auf ein Bandmagazin als Erzeugnis wird die eingangs genannte Aufgabe dadurch gelöst, dass ein auf einer Spule aufwickelbares oder aufgewickeltes Trägerband und eine Vielzahl von auf dem Trägerband angeordnete disposible Lanzetten vorgesehen sind, wobei die Lanzetten in jeweils einer vorgefertigten bzw. gesonderten Packung angeordnet sind, und wobei die Lanzetten-Packungen im Abstand voneinander auf dem Trägerband fixiert sind.

Gegenstand der Erfindung ist auch ein Bandmagazin zur Bevorratung von Lanzetten und Testelementen insbesondere für Blutglucoseuntersuchungen, wobei die Lanzetten und Testelemente in jeweils einer vorgefertigten Packungseinheit angeordnet sind, und wobei die Lanzetten- und Testelement-Packungseinheiten vorzugsweise alternierend im Abstand voneinander auf dem Trägerband fixiert sind.

Im Folgenden wird die Erfindung anhand der in der Zeichnung schematisch dargestellten Ausführungsbeispiele näher erläutert. Es zeigen
- Fig. 1: ein Lanzettenband in einer abgebrochenen perspektivischen Darstellung;
- Fig. 2: eine Ausschnittsvergrößerung des Lanzettenbands nach Fig. 1;
- Fig. 3: den Schichtaufbau des Lanzettenbands in einer Explosionsdarstellung;
- Fig. 4: und 5 einen Schnitt durch das Lanzettenband in Bandquerrichtung und Bandlängsrichtung;
- Fig. 6: und 7 eine schaubildliche Darstellung verschiedener Schritte zur Fertigung von Lanzetten-Packungen;
- Fig. 8: einen Stempel zum Heißsiegeln der Lanzetten-Packungen in der Draufsicht;
- Fig. 9: einen Schnitt entlang der Linie 9 - 9 der Fig. 8;
- Fig. 10: eine Bandkassette mit einem mit Lanzetten und Testelementen bestückten Testband in teilweise aufgebrochener perspektivischer Darstellung;
- Fig. 11 und 12: die Ingebrauchnahme einer Lanzette in perspektivischer und geschnittener Ansicht.

Die in der Zeichnung dargestellten Lanzettenvorratsbänder 10 ermöglichen die Magazinierung bzw. Bereitstellung einer Vielzahl von Lanzetten 12 als Einmalartikel für Blutglucosebestimmungen oder andere Untersuchungen, bei denen durch einen Hauteinstich eine Körperflüssigkeitsprobe für diagnostische Zwecke gewonnen werden soll. Daneben sind auch Kombinationen von Lanzetten und diagnostischen Testelementen auf einem gemeinsamen Vorratsband möglich. In beiden Fällen werden vorgefertigte Packungs- oder Trägereinheiten bereitgestellt, die auf einem Trägerband in Form eines Bandwickels magaziniert werden.

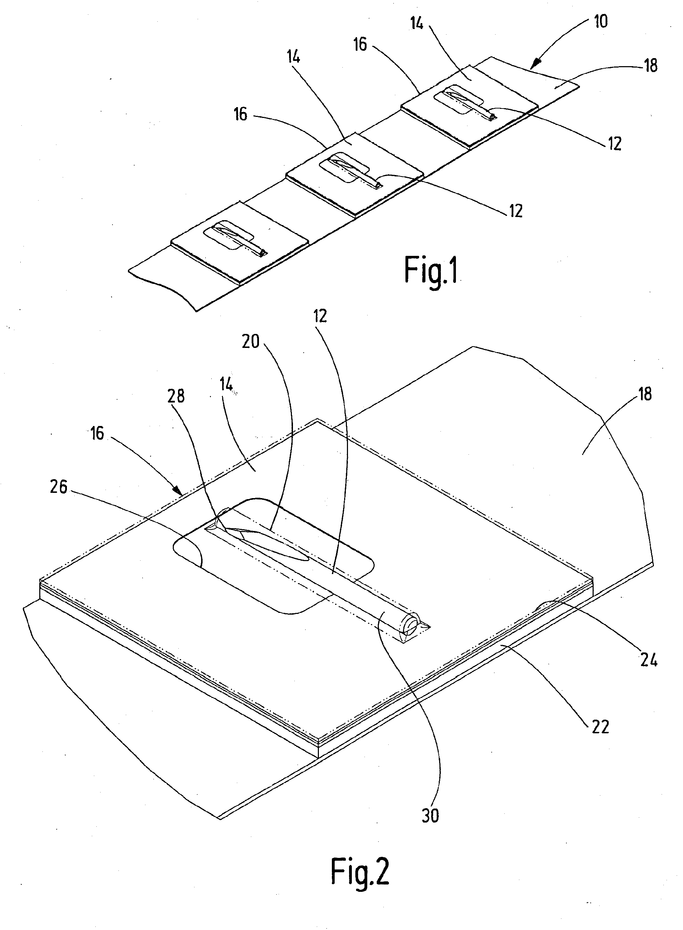

Wie in Fig. 1 gezeigt, sind die Lanzetten 12 in jeweils einer zugeordneten Packung 14 angeordnet, wobei die so gebildeten Verpackungseinheiten bzw. Lanzetten-Packungen 16 im Abstand voneinander nach Art von Etiketten auf dem Trägerband 18 fixiert sind. Aufgrund der flexiblen und flachen Lanzetten-Packungen 16 ergibt sich so ein aufrollbares Bandmagazin, das in ein nicht gezeigtes Handgerät zur automatischen Handhabung einsetzbar ist.

In der Vergrößerung nach Fig. 2 ist zu ersehen, dass die jeweilige Lanzette 12 in einer durch die Packung 14 gebildeten Tasche 20 geschützt ist. Die Tasche 20 wird durch einen Folienverbund aus einer Bodenfolie 22 und einer Deckfolie 24 gebildet. Ein erweiterter Taschenbereich 26 nimmt die Lanzettenspitze 28 freiliegend auf, während der proximale Schaftabschnitt 30 der Lanzette 12 eng eingeschlossen wird. Ein maschineller Umgang auch mit sehr kleinen Nadelelementen wird somit erleichtert, ohne dass eine Beschädigung der sehr empfindlichen Spitze sowie eine Beeinträchtigung von deren Sterilität befürchtet werden muss. In der gezeigten Ausführung ist eine in Bandquerrichtung ausgerichtete Rundlanzette vorgesehen. Denkbar sind auch andere Orientierungen und Ausformungen, beispielsweise in Form eines mit einem rillenförmigen kapillaren Sammelkanal versehenen flachen Stechelements.

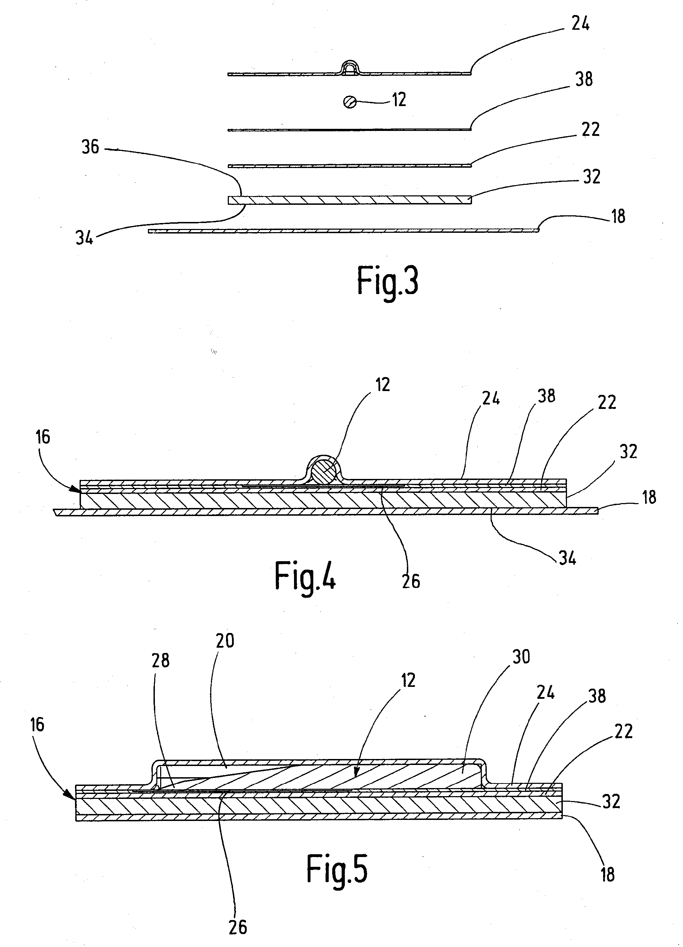

Fig. 3 zeigt die einzelnen Bestandteile des Folienaufbaus nach Fig. 2. Als Trägerband 18 kann beispielsweise eine ca. 10 µm dicke und ca. 5 - 10 mm breite PET-Folie dienen. Die Verbindung der etikettenartigen Lanzetten-Packung 16 vermittelt ein Abschnitt eines doppelseitigen Klebebands 32, welches eine erste Klebefläche 34 für das Trägerband 18 und eine gegenüberliegende zweite Klebefläche 36 für die Bodenfolie 22 besitzt. Alternativ lässt sich auch ein einseitig klebendes Klebebandstück einsetzen, das zugleich die Bodenfolie bildet. Anstelle einer Klebeverbindung ist auch eine sonstige stoffschlüssige oder mechanische Verbindung (z.B. Klettverschluss) denkbar. Der Verbund zwischen Bodenfolie 22 und Deckfolie 24 unter Einschluss der Lanzette 12 erfolgt über eine bodenfolienseitige Schmelzkleberschicht 38, die durch einen Heißstempel aktiviert wird, wie nachstehend näher erläutert.

Fig. 4 und 5 zeigen das Fertigprodukt nach Fig. 2 in einem Schnitt quer und längs zu der Lanzette 12. Die dünne Deckfolie schmiegt sich über den Lanzettenquerschnitt an, so dass eine Versiegelung rund um die Lanzette 12 erfolgt. Dabei ist auch zu ersehen, dass der proximale Lanzettenabschnitt 30 über die Schmelzkleberschicht 38 auf der Bodenfolie 22 fixiert ist, während der distale Lanzettenabschnitt 28 in dem erweiterten unverschweißten Bereich 26 der Tasche 20 für die Benutzung freigehalten wird. Vor allem in der gezeigten Querausrichtung der Lanzetten 12 quer zur Bandlängsrichtung ist es zweckmäßig, wenn die Lanzetten-Packungen 16 unter Berücksichtigung von Fertigungstoleranzen entsprechend der Breite des Trägerbandes 18 dimensioniert werden.

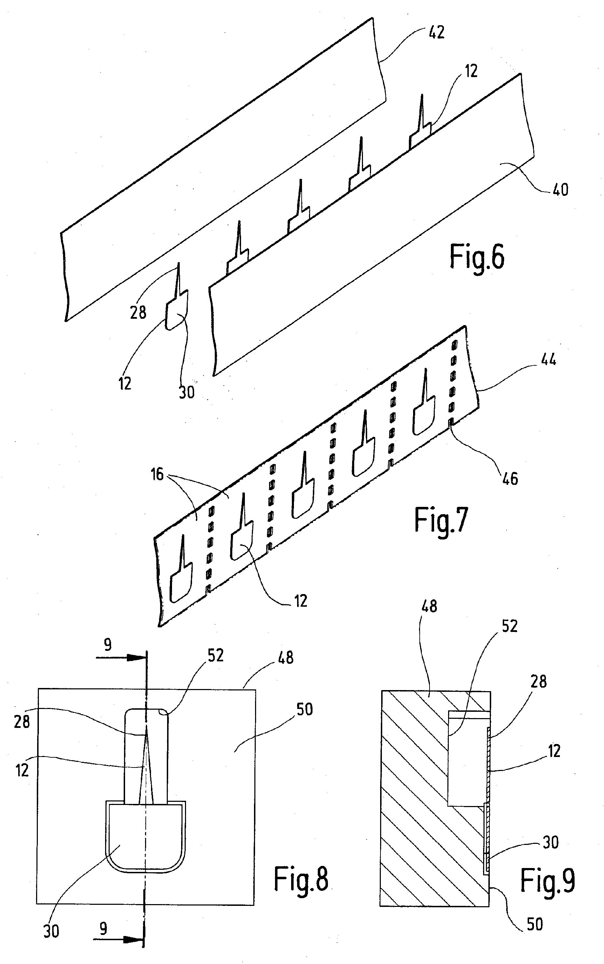

Fig. 6 veranschaulicht die Vorfertigung eines Packungsbandes mit einer Vielzahl von zusammenhängenden Lanzetten-Packungen. Zu diesem Zweck werden ein Bodenfolienstreifen 40 und ein Deckfolienstreifen 42 unter Einschluss der Lanzetten - hier Flachlanzetten 12 mit einer Lanzettenspitze 28 und einem flächigen proximalen Halterteil 30 - zusammengeführt und fest miteinander verbunden. Als Bodenfolienstreifen 40 kann beispielsweise eine etwa 20 µm dicke PET-Folie eingesetzt werden, die einseitig mit einem Heißkleber aus einem niedrigschmelzenden Polyester, z.B. einem Ethylenvinylacetat-Copolymer beschichtet ist. Der Abdeckfolienstreifen 42 ist zweckmäßig dünner als die Bodenfolienstreifen 40 ausgebildet, beispielsweise als 5 µm dicke PET-Folie.

Wie in Fig. 7 gezeigt, wird das Packungsband 44 mit quer verlaufenden Perforationen 46 versehen, die eine Vereinzelung der Lanzetten-Packungen 16 vor dem Aufbringen auf das Trägerband 18 vereinfachen. Möglich ist es auch, die einzelnen Packungen durch heiße Schneidmittel zugleich zu zerteilen und entlang der Schnittlinie zu verschweißen, wobei thermoplastisches Folienmaterial eingesetzt wird. Eine weitere Variante sieht vor, dass ein längs gefalteter Folienstreifen mit Lanzetten bestückt und quer in einzelne Packungen zerteilt wird (nicht gezeigt). Hierbei bildet eine Längshälfte des Folienstreifens den Bodenbereich zum Aufbringen der Lanzetten, während die andere Längshälfte anschließend übergefaltet und mit dem Bodenbereich verbunden wird.

Fig. 8 und 9 zeigen die Versiegelung der einzelnen Lanzetten-Packungen durch einen auf den Schmelzkleber einwirkenden Heißstempel 48. Dieser ist an seiner auf die jeweilige Lanzetten-Packung 16 angedrückten Stempelfläche 50 mit einer abgestuften Aussparung 52 versehen, welche den proximalen Nadelabschnitt 30 eng umschließt und durch eine entsprechende Vertiefung für den erweiterten unverschweißten Bereich 26 der Tasche 20 im Bereich der Nadelspitze 28 sorgt. Hierbei wird der proximale Nadelabschnitt 30 bevorzugt mit der Bodenfolie 22 fest verklebt, so dass die Nadel 12 nach einer späteren Verwendung in Verbindung mit dem Trägerband 18 remagaziniert werden kann.

Wie nicht eigens dargestellt, können die in den Lanzetten-Packungen 16 eingeschweißten Lanzetten 12 durch Bestrahlung beispielsweise mit einem hochenergetischen Elektronenstrahl sterilisiert werden. Aufgrund der stoffdichten Versiegelung ist die Sterilhaltung auch im weiteren Verarbeitungsablauf gewährleistet.

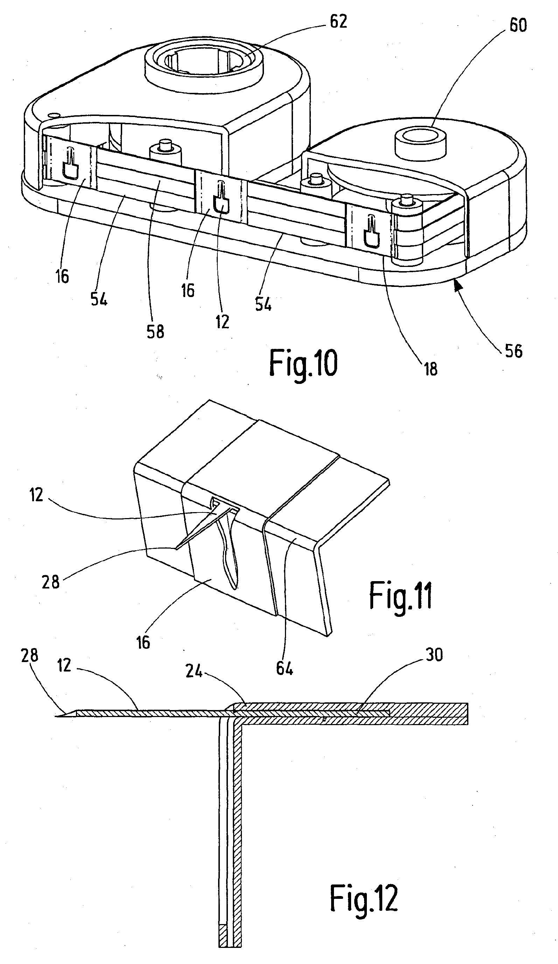

Die Lanzetten-Packungen 16 werden an einer Etikettierstation auf das Trägerband 18 aufetikettiert. Hierbei können auch zusätzlich Testelemente in Form von Testfeld-Etiketten 54 aufgebracht werden, wie es in Fig. 10 am Beispiel einer Bandkassette 56 ersichtlich ist. Die Testfeld-Etiketten 54 sind mit einer Reagenzschicht 58 versehen, die mit durch einen Hauteinstich gewonnener Körperflüssigkeit (Blut und/oder Gewebeflüssigkeit) beaufschlagbar ist, um einen Analyten (Glucose) nachzuweisen. Der Nachweis erfolgt vorzugsweise durch eine Farbänderung, kann aber auch elektrochemischer Natur sein. Die maschinelle Handhabung der unterschiedlichen disposiblen Einheiten 16, 54 an der Etikettierstation kann somit in einem einheitlichen Etikettier-Prozess erfolgen, wie er nur für Testfeld-Etiketten in der EP-A 1 593 434 beschrieben ist, worauf hier Bezug genommen wird.

Die Kassette 56 besitzt eine Vorratsspule 60 für unverbrauchtes Bandmaterial und eine Aufwickelspule 62 zur Remagazinierung bzw. Entsorgung von verbrauchten Einheiten 16, 54. Die Bereitstellung erfolgt durch sukzessiven Bandvorschub vorzugsweise in einem Handgerät, um einen weitgehend automatischen Messablauf zu ermöglichen.

Im Zuge einer solchen Messung, die vom Patienten vor Ort selbst vorgenommen werden kann, wird die jeweilige Lanzette 12 gemäß Fig. 11 und 12 zum Einsatz gebracht. Durch Knicken der Lanzetten-Packung 16 an einer Umlenkstelle 64 wird die dünne Deckfolie 24 von der Lanzette 12 aufgeschlitzt und dabei die Lanzettenspitze 28 freigelegt. Durch einen geeigneten Aktuator kann dann eine Stechbewegung beispielsweise zum Einstechen in einen Finger durchgeführt werden. Der proximale Lanzettenabschnitt 30 bleibt hierbei zweckmäßig mit dem Folienlaminat verbunden, so dass die anschließende Entsorgung der Lanzette 12 auf dem Trägerband 18 vereinfacht wird.

## Patentansprüche

1. Verfahren zur Magazinierung von Lanzetten insbesondere für Blutglucoseuntersuchungen bei dem eine Vielzahl von mit einer Lanzettenspitze (28) zum Einstechen in die Haut versehenen disposiblen Lanzetten (12) auf einem auf einer Spule (60,62) aufgewickelten oder einem aufwickelbaren Trägerband (18) zum Gewinnen einer Körperflüssigkeitsprobe bereitgestellt werden, **dadurch gekennzeichnet, dass** die Lanzetten (12) in jeweils einer Packung (14) angeordnet werden, und dass die so gebildeten Lanzetten-Packungen (16) einzeln auf das Trägerband (18) aufgebracht und darauf fixiert werden.

2. Verfahren nach Anspruch 1, **dadurch gekennzeichnet, dass** die Packungen (14) aus einer Bodenfolie (22) und einer damit verbundenen Deckfolie (24) gebildet werden.

3. Verfahren nach Anspruch 2, **dadurch gekennzeichnet, dass** die Bodenfolie (22) und die Deckfolie (24) unter Einschluss der Lanzetten (12) miteinander in Kontakt gebracht und verbunden werden.

4. Verfahren nach Anspruch 2 oder 3, **dadurch gekennzeichnet, dass** die Bodenfolie (22) und die Deckfolie (24) über eine Schmelzkleberverbindung miteinander verbunden werden, wobei eine vorzugsweise auf der Bodenfolie (22) befindliche Schmelzkleberschicht (38) durch einen Heißstempel (48) aktiviert wird.

5. Verfahren nach einem der Ansprüche 1 bis 4, **dadurch gekennzeichnet, dass** eine Vielzahl von Lanzetten-Packungen (16) als Packungsband (44) zusammenhängend gefertigt und vor dem Aufbringen auf das Trägerband (18) vereinzelt wird.

6. Verfahren nach Anspruch 5, **dadurch gekennzeichnet, dass** das Packungsband (44) durch Zusammenführen und Verbinden eines Bodenfolienstreifens (40) und eines Deckfolienstreifens (42) gebildet wird.

7. Verfahren nach Anspruch 5, **dadurch gekennzeichnet, dass** das Packungsband (44) durch Umklappen eines Folienstreifens und Bildung von Lanzettentaschen in dem gefalteten Folienstreifen gefertigt wird.

8. Verfahren nach einem der Ansprüche 1 bis 7, **dadurch gekennzeichnet, dass** die Lanzetten-Packungen (16) durch einen Heißschnitt zugleich zugeschnitten und unter Einschluss der Lanzetten (12) dicht umrandet werden.

9. Verfahren nach einem der Ansprüche 1 bis 8, **dadurch gekennzeichnet, dass** die Lanzetten (12) vorzugsweise an einem proximalen Endabschnitt (30) fest mit der Packung (14) verbunden werden.

10. Verfahren nach einem der Ansprüche 1 bis 9, **dadurch gekennzeichnet, dass** zumindest Spitzen (28) der Lanzetten (12) in der Packung (14) gegen Beschädigung geschützt und/oder steril gehalten sind.

11. Verfahren nach einem der Ansprüche 1 bis 10, **dadurch gekennzeichnet, dass** die Lanzettenspitzen (28) in einer durch die Packung (14) gebildeten Tasche (20) angeordnet werden.

12. Verfahren nach Anspruch 1, **dadurch gekennzeichnet, dass** die Lanzetten-Packungen (16) durch eine Bodenfolie (22) und eine darauf aufgebrachte und zumindest die Lanzettenspitze (28) umschließende, insbesondere aus Silikon bestehende Verschlussmasse gebildet werden.

13. Verfahren nach einem der Ansprüche 1 bis 12, **dadurch gekennzeichnet, dass** zumindest die Spitzen (28) der Lanzetten (12) in den Lanzetten-Packungen (16) vor dem Aufbringen auf das Trägerband (18) vorzugsweise durch hochenergetische Bestrahlung sterilisiert werden.

14. Verfahren nach einem der Ansprüche 1 bis 13, **dadurch gekennzeichnet, dass** die Lanzetten-Packungen (16) jeweils an einer von der Lanzette (12) abgewandten Verbindungsfläche (34) auf dem Trägerband (18) fixiert werden.

15. Verfahren nach einem der Ansprüche 1 bis 14, **dadurch gekennzeichnet, dass** die Lanzetten-Packungen (16) in Form von Etiketten oder Aufklebern auf das Trägerband (18) aufgebracht werden.

16. Verfahren nach einem der Ansprüche 1 bis 15, **dadurch gekennzeichnet, dass** die Lanzetten-Packungen (16) mit einem Klebebandstück (32) zum Aufkleben auf das Trägerband (18) versehen werden.

17. Verfahren nach einem der Ansprüche 1 bis 16, **dadurch gekennzeichnet, dass** die Lanzetten-Packungen (16) durch mechanische Verbindungsmittel oder durch Verschweißen auf dem Trägerband (18) gehalten werden.

18. Verfahren nach einem der Ansprüche 1 bis 17, **dadurch gekennzeichnet, dass** auf dem Trägerband (18) vorzugsweise alternierend zu den Lanzetten-Packungen (16) mit der Köperflüssigkeitsprobe beaufschlagbare Testelemente (54) aufgebracht werden.

19. Verfahren zur Magazinierung von Lanzetten und Testelementen (54) insbesondere für Blutglucoseuntersuchungen bei dem eine Vielzahl von in die Haut einstechbaren Lanzetten (12) und mit Körperflüssigkeit beaufschlagbaren Testelementen (54) auf einem auf einer Spule (60,62) aufgewickelten oder einem aufwickelbaren Trägerband (18) zum Untersuchen einer Körperflüssigkeitsprobe bereitgestellt werden, **dadurch gekennzeichnet, dass** die Lanzetten (12) und die Testelemente (54) gesondert als Packungseinheiten vorgefertigt werden, und dass anschließend in einem einheitlichen Bestückungsprozess die Packungseinheiten auf das Trägerband (18) aufgebracht und darauf fixiert werden.

20. Verfahren nach Anspruch 18 oder 19, **dadurch gekennzeichnet, dass** die flachen Lanzetten- und Testelement-Packungseinheiten eine im Wesentlichen übereinstimmende, vorzugsweise der Breite des Trägerbands (18) entsprechende Breite aufweisen.

21. Verfahren nach einem der Ansprüche 1 bis 20, **dadurch gekennzeichnet, dass** das Trägerband (18) in einer Kassette (56) angeordnet wird, so dass es von einer Abwickelspule (60) abziehbar und auf eine Aufwickelspule (62) aufspulbar ist.

22. Bandmagazin zur Bevorratung von Lanzetten insbesondere für Blutglucoseuntersuchungen mit einem auf einer Spule (60,62) aufwickelbaren oder aufgewickelten Trägerband (18) und einer Vielzahl von auf dem Trägerband (18) angeordneten disposiblen Lanzetten (12), die mit einer Lanzettenspitze (28) zum Einstechen in die Haut versehen sind, **dadurch gekennzeichnet, dass** die Lanzetten (12) in jeweils einer vorgefertigten Packung (14) angeordnet sind, und dass die Lanzetten-Packungen (16) im Abstand voneinander auf dem Trägerband (18) fixiert sind.

23. Bandmagazin zur Bevorratung von Lanzetten und Testelementen (54) insbesondere für Blutglucoseuntersuchungen mit einem auf einer Spule (60,62) aufgewickelten oder aufwickelbaren Trägerband (18) und einer Vielzahl von auf dem Trägerband (18) angeordneten disposiblen Lanzetten (12) und Testelementen (54), **dadurch gekennzeichnet, dass** die Lanzetten (12) und Testelemente (54) in jeweils einer vorgefertigten Packungseinheit angeordnet sind, und dass die Lanzetten- und Testelement-Packungseinheiten vorzugsweise alternierend im Abstand voneinander auf dem Trägerband (18) fixiert sind.
